# EUROPEAN PATENT SPECIFICATION

(11) **EP 4 017 441 B1**
(45) Date of publication and mention of the grant of the patent: **04.03.2026**
(21) Application number: 20853622.7
(22) Date of filing: 21.08.2020
(51) Int. Cl.: A61F 9/013, A61B 17/32, A61F 9/011, A61F 9/007

(54) **OPHTHALMIC BLADES**
OPHTHALMISCHE KLINGEN
INSTRUMENTS CHIRURGICAUX OPHTALMIQUES

(30) Priority: 21.08.2019 AU 2019903050
(43) Date of publication of application: 29.06.2022
(73) Proprietor: Kuo, Chih-Hung, Garran, ACT 2605 (AU)
(72) Inventor: Kuo, Chih-Hung, Garran, ACT 2605 (AU)
(74) Representative: Sweetinburgh, Mark Roger
(86) International application number: PCT/AU2020/050871
(87) International publication number: WO 2021/030873

(56) References cited:
- WO-A1-2004/112665
- WO-A1-2017/039463
- JP-A- 2005 334 054
- JP-A- 2013 150 675
- US-A- 5 217 477
- US-A- 5 224 950
- US-A1- 2005 033 335
- US-A1- 2006 058 824
- US-A1- 2008 215 078
- US-A1- 2009 062 810

## Description

### TECHNICAL FIELD

The present invention in one form relates to a multi-purpose ophthalmic blade .

### BACKGROUND

There are many different sized and shaped surgical blades for ophthalmic surgeries. In the course of a surgical procedure, an ophthalmologist may typically use two or more different blade-types.

For example, during routine cataract extraction surgery, an ophthalmologist typically uses a paracentesis or side port blade for making a side port incision and a keratome or slit blade for making a primary clear corneal incision. Accordingly, during such a procedure the ophthalmologist hands over the side port blade after making the side port incision before being handed the next instrument for making the primary clear corneal incision or injecting viscoelastic material.

The procedure is further complicated by the timely need for injection of the viscoelastic material via the side port or primary incision to maintain the anterior chamber depth and protect the corneal endothelial cells. Generally, the material is injected to deepen the anterior chamber of the eye and has to be timely injected before the anterior chamber collapses due to fluid loss during one or both of the incisions, especially for surgeons in their early training. Practice varies with some ophthalmologists injecting the viscoelastic material in between making incisions and other ophthalmologists injecting the viscoelastic material after making both incisions.

A problem in general with the above practice is that the multiple instrument handling delays the procedure thereby unnecessarily exposing a patient to a greater risk surgical complication, particularly when the ophthalmologist is rushing to apply the viscoelastic material after, or in between, making the incisions in a collapsed anterior chamber.

Another related problem is the risk of needle stick-type injuries occurring for both the ophthalmologist and support staff due to the multiple instrument handling.

Yet further problems with the above practice are the costs and waste involved in stocking and supplying multiple instruments for each procedure, particularly since the instruments are generally single-use items.

In contrast to the above practice, some more experienced ophthalmologists may form the two corneal incisions using a single keratome blade. However, such an alternate practice is also not without problems. For example, the formation of the two corneal incisions with a single keratome blade may cause anterior chamber instability due to the formation of an enlarged side port incision and may also result in a less predictable refractive outcome.

Earlier instruments have been developed to attempt to reduce the number of instruments required for a procedure.

For example, International Patent Publication No. WO 2004/112665 A discloses a = blade for making and then widening a single incision for cataract removal surgery. However, the blade is not suitable for, nor applicable to, modern cataract extraction surgeries, which require the making and simultaneous use of more than one incision. Further, the disclosed blade would be prone to over extension into the anterior chamber of the eye.

Likewise, US Patent No. 5,217,477 US5224950) and US2008/215078 disclose a blade for making and then widening a single incision for phacoemulsification and then IOL implantation. However, again the inventor has recognised that the blade is not suitable for, nor applicable to, modern cataract extraction surgeries, which require the making of multiple precise incisions. The disclosed blade would be prone to forming enlarged side port incisions.

It will be clearly understood that, if a prior art publication is referred to herein, this reference does not constitute an admission that the publication forms part of the common general knowledge in the art in Australia or in any other country.

### SUMMARY OF INVENTION

Embodiments of the present invention provide a multi-purpose ophthalmic blade, which may at least partially address one or more of the problems or deficiencies mentioned above or which may provide the public with a useful or commercial choice.

According to a first aspect of the present invention, there is provided a multi-purpose ophthalmic blade adapted for cataract extraction surgery, said blade including:
a shaft connectable to a handle;
a first blade portion extending from the shaft along a central axis, said first blade portion including a pair of opposed side edge portions extending about the central axis, the side edge portions defining a first pair of opposed cutting edges each oriented at a first angle relative to said central axis and that at least partially converge towards a distal end of the first blade portion; and
a second blade portion extending distally forward from the first blade portion along the central axis to a blade tip, said second blade portion including a pair of opposed parallel side edges portions without cutting edges defined thereon, and a pair of converging side edge portions extending distally forward from the parallel side edge portions, said converging side edge portions defining a second pair of cutting edges that converge to the blade tip, said second pair of cutting edges each being oriented at a second angle relative to said central axis,
wherein said first blade portion is adapted for making a primary incision and said second blade portion is adapted for making a side port incision.

Typically, wherein the second blade portion extends distally forward from the first blade a distance equal to or less than a multiple of 1.2 times a width of the second blade portion at its widest point, preferably a multiple of 1.1 times, more preferably a multiple of 1.0 times.

Advantageously, the multi-purpose blade of the present invention enables an ophthalmologist to be more efficient during a surgical procedure by reducing the number of instruments required. In turn, the reduction in instrument handling advantageously speeds up surgical procedures thereby reducing the risk of infection, surgical complications and needle stick-type injuries. Lastly, by reducing the number of surgical instruments required, the present invention reduces waste and provides a cost-effective alternative to current practice.

As used herein, the term "eye" refers to a human eye.

The eye is not a perfect sphere but rather is a fused two-piece unit. The eye includes a smaller frontal unit called the "cornea", which is linked to a larger white unit called the "sclera". The cornea is transparent and is more curved than the sclera.

As used herein, the term "corneoscleral junction" or "limbus" refers to a border of the cornea and the sclera, or the transition region between the cornea and the sclera.

The eye further includes a coloured circular structure called the "iris" located within the sclera. The iris concentrically surrounds a pupil of the eye, which appears to be black. The size of the pupil, which controls the amount of light entering the eye, is adjusted by the iris' dilator and sphincter muscles.

Light enters the eye through the cornea, then the pupil and then through a lens controlled by ciliary muscles. The light then falls on light-sensitive cells located at the back of the eye called the "retina". The light-sensitive cells of the retina convert the light into electrical signals that are carried to the brain by the optic nerves.

The lens is a transparent, biconvex structure that, along with the cornea, helps to refract light to be focussed on the retina.

As used herein, the term "cataract" refers to an opacification of the lens in the eye causing an impairment or loss of vision. Metabolic changes of the lens fibres over time lead to the development of a cataract, which reduces the transmission of light to the retina. Cataract extraction surgery generally involves removal and replacement of the lens with an artificial lens also known as an intraocular lens (IOL).

As used herein, the term "clear corneal incision" or "corneal incision" refers to an incision usually made linear or curvilinear to the limbus and that dissects anteriorly into the cornea.

As used herein, the term "primary clear corneal incision" or "primary incision" generally refers to a corneal incision made for emulsification and aspiration of the natural lens and implantation of the artificial lens as part of a cataract extraction surgery. Generally, the incision is of a size to be self-sealing. Usually, the incision is between about 2.0mm and about 4.0 mm in length and about 2-3mm in width, typically between about 2.2mm and about 2.75mm in width.

As used herein, the terms "secondary clear corneal incision" or "side port incision" refer to a corneal incision made for delivery and removal of viscoelastic material as well as for general manipulation of the natural lens and implanted artificial lens during the course of a surgical procedure. Again, generally the incision is of a size to be self-sealing. Usually, the incision is between about 1.0mm in length and between about 0.6mm and about 1.5mm in width.

As used herein, the term "viscoelastic material" refers to a flowable substance that is delivered and removed from the anterior chamber of an eye to prevent corneal endothelial cell loss, maintain eye pressurization/anterior chamber depth and/or distend the lens' capsule during the course of a surgical procedure. The substance is also used to coat the endothelium, absorb ultrasound energy and mechanically protect the endothelium against surgical trauma.

As indicated above, the present invention provides a multi-purpose ophthalmic blade for use in cataract extraction surgery. The blade may be of any size, shape and construction as long as falling under the definition of independent claim 1, and may be formed from any material or materials suitably configured for ophthalmic surgery, preferably cataract extraction surgery.

Generally, the blade may be formed from any material or materials capable of forming a sharp cutting edge. Usually, the blade may be formed from metal and/or plastic material or materials, preferably stainless steel, titanium or aluminium. Although, in some embodiments, it is envisaged that the blade or at least the cutting edges may be formed from diamond. Preferably, the blade may be integrally formed.

The shaft may be of any suitable size, shape and length for spacing the blade portions relative to the handle.

Generally, the shaft may include a pair of opposed ends and may extend longitudinally between the opposed ends. The opposed ends may include a proximal end connectable to a handle and an opposed distal end from which the first blade portion extends.

The shaft may be of tubular or solid construction, typically solid.

The shaft may have any suitable cross-sectional shape. For example, the shaft may have a substantially rectangular, triangular, pentagonal, hexagonal, heptagonal, octagonal, circular, or oval shaped cross-section. Preferably, the shaft may have a substantially circular cross section at or near the proximal end and may taper or flatten at or near the distal end from which the first blade portion extends.

The shaft may extend in a linear direction between the handle and the first blade portion.

The shaft may include one or more bends. For example, the shaft may include a distal end portion and a proximal end portion angled relative to one another. It is envisaged that the distal end portion may extend at angle ranging between about 120° and about 150° relative to the proximal end portion, preferably at an angle ranging between about 130° and about 140°, more preferably at an angle of about 135°.

As indicated, the shaft may be connectable to a handle via the proximal end. The handle may typically have an elongate shape suitable for being held and manipulated by a hand of an ophthalmologist. The handle may include an elongate body extending longitudinally between two opposed ends in a linear direction.

The handle may be formed from metal and/or plastic material or materials, typically plastic materials.

The shaft and the handle may be connectable in any suitable way known in the art. For example, in some embodiments in which the blade is re-usable, the shaft may be releasably connectable to the handle via a connecting mechanism. The shaft may be permanently connected to the handle, preferably integrally connected. For example, the handle may be formed over the proximal end of the shaft, preferably overmoulded.

The shaft may extend any suitable length between a connected handle and the blade portions. For example, the shaft may extend about 5mm, about 6mm, about 7mm, about 8mm, about 9mm, about 10mm, about 11mm, about 12mm, about 13mm, about 14mm, about 15mm, about 16mm, about 17mm, about 18mm, about 19mm or even about 20mm between the handle and the blade portions. Typically, the shaft may have a length of between about 6mm and about 12mm extending between a connected handle and the blade portions, preferably the shaft may extend a length of about 9mm between a connected handle and the blade portions.

As indicated, the shaft may taper or flatten at or near the distal end from which the first blade portion extends, preferably flatten such that the first blade portion (and the second blade portion) may extend at an angle relative to the shaft. For example, the first blade portion may extend at an angle relative to the shaft ranging between about 120° and about 150°, preferably at an angle ranging between about 130° and about 140°, more preferably at an angle of about 135°.

The blade may include a body. The body may define the first and second blade portions.

The body may be of any suitable size, shape and construction to define the first blade portion and the second blade portion.

Typically, the body may extend from the distal end of the shaft along a central axis to the blade tip, preferably a pointed blade tip.

Generally, the body may be in the form of a plate. The body may be substantially flat or planar and may have a polygonal shape. In some embodiments, the body may have a substantially diamond or kite shape. Z The body may have a substantially trapezoidal shape.

The body may have two opposed surfaces, including an upper surface that, during surgery, faces outwards of an eye, and an opposed lower surface. The opposed surfaces may extend substantially parallel to one another and be interconnected by opposing edges, including opposed side edges, a proximal end edge from which the body (and the first blade portion) extends from the shaft, and an opposed distal end edge defining the blade tip of the second blade portion.

The body may define the first blade portion at or near a proximal end of the body, the second blade portion at or near the distal end of the body and a junction between the first and second blade portions.

As indicated, the first blade portion (and the second blade portion) may extend from the shaft along the central axis. The first blade portion may include a pair of opposed side edge portions that extends about the central axis, preferably symmetrically.

The first pair of opposed cutting edges may be defined on at least a portion of the opposed side edge portions. The side edge portions may correspond to portions of the side edges of the blade body.

Generally, one or both said side edge portions of the first blade body may flare and taper respectively away and at least partially towards the central axis as they extend between the proximal end and the junction to partly define the shape of the blade and the blade portions. The side edge portions may flare and taper in a linear or curvilinear direction, or a combination thereof.

For example, one or both side edge portions, as they extend between the proximal end and the junction, may flare outwards to define a widest part, also known as the apex, of the blade and then taper back at or near the junction. The widest part of the blade may be a distinct point. The widest part of the blade may be a portion of the both side edge potions extending substantially parallel to one another.

The apex may define proximal end facing side edge portions extending between the proximal end and the apex and distal-end facing side edge portions extending between the apex and the junction, and preferably having the first pair of cutting edges defined thereon.

In preferred embodiments, both side edge portions of the first blade portion may gently flare outwards and away from the central axis as they extend along from the proximal end at least partially towards the junction to define the apex of the blade. The side edge portions may gently taper at least partially towards the central axis as they extend from the apex towards the junction of the first blade portion to define the distal-end facing side edge portions.

The first pair of cutting edges may be defined on the distal-end facing side edge portions of the first blade portion such that the first cutting edges form an incision as the blade is advanced into the eye in a direction parallel with the central axis. Typically, the proximal end facing side edge portions have rounded or non-cutting edges so as not to modify the incision, i.e., enlarge the incision or cause superficial cuts, as the blade is retracted.

Generally, the distal-end facing side edge portions may taper at least partially towards the central in a linear direction, although a curvilinear direction is also envisaged.

As indicated, the second blade portion may extend distally forward from the first blade portion along the central axis to the blade tip. Like the first blade portion, the second blade portion may include a pair of opposed side edge portions that at least partially converge to the blade tip. The second pair of cutting edges may be defined on at least a portion of the side edge portions.

The opposed side edge portions of the second blade portion may extend about the central axis, preferably symmetrically.

The pair of opposed side edge portions may at least partially converge to the blade tip in a linear direction, although a curvilinear direction is also envisaged.

Again, the side edge portions may correspond to portions of the side edges of the body.

The second blade portion includes a pair of opposed substantially parallel side edge portions and a pair of converging side edge portions extending distally forward from the opposed substantially parallel side edge portions. The second pair of cutting edges is defined on the pair of converging side edge portions and converges to the blade tip. The cutting edges are not be defined on the substantially parallel side edge portions.

Advantageously, the substantially parallel side edge portions facilitate in the forming of side port incisions of precise width, wherein the width is defined by a maximum width of the second blade portion, i.e., as measured between the substantially parallel side edge portions. Further, the absence of cutting edges on the substantially parallel side edge portions, ensures that the side port incision is not inadvertently enlarged as the blade is advanced into the eye in a direction parallel with the central axis up to and not beyond the substantially parallel side edge portions of the second blade portion.

The substantially parallel side edge portions may define, or at least partially define, the junction between the first and second blade portions.

The first and second pairs of cutting edges are separated from one another, e.g., by the substantially parallel side edge portions of the second blade portion.

Generally, the respective cutting edge pairs may be oriented at different angles relative to the central axis. However, each pair of cutting edges may be oriented relative to the central axis such that they face, or at least partially face towards the blade tip to facilitate incision formation when the blade is advanced into the eye in a direction parallel with the central axis.

In some embodiments, the first pair of cutting edges may be oriented at a first angle relative to the central axis that is greater than the second angle at which second pair of cutting edges are oriented relative to the central axis. In other embodiments, the first angle may be less than the second angle. In yet other embodiments, the first angle and the second angle may be substantially the same.

The first angle, as measured between each of the first pair of cutting edges and the central axis, may be about 15°, about 16°, about 17°, about 18°, about 19°, about 20°, about 21°, about 22°, about 23°, about 24°, about 25°, about 26°, about 27°, about 28°, about 29°, about 30°, about 31°, about 32°, about 33°, about 34°, about 35°, about 36°, about 37°, about 38°, about 39° or even about 40°, typically between about 18° and about 35°, preferably between about 25° and about 35°.

The first pair of cutting edges may have a maximum width extending between the opposed cutting edges, as measured at or near the apex, of about 1.0mm, about 1.1mm, about 1.2mm, about 1.2mm, about 1.3mm, about 1.4mm, about 1.5mm, about 1.6mm, about 1.7mm, about 1.8mm, about 1.9mm, about 2.0mm, about 2.1mm, about 2.2mm, about 2.3mm, about 2.4mm, about 2.5mm, about 2.6mm, about 2.7mm, about 2.8mm, about 2.9mm, about 3.0mm, about 3.1mm, about 3.2mm, about 3.3mm, about 3.4mm or even about 3.5mm, typically ranging between about 1.3mm and 3.2mm, preferably between about 2.0mm and about 2.2mm.

In contrast, the second angle may be about 5°, about 6°, about 7°, about 8°, about 9°, about 10°, about 11°, about 12°, about 13°, about 14°, about 15°, about 16°, about 17°, about 18°, about 19°, about 20°, about 21°, about 22°, about 23°, about 24°, about 25°, about 26°, about 27°, about 28°, about 29°, about 30°, about 31°, about 32°, about 33°, about 34°, about 35°, about 36°, about 37°, about 38°, about 39°, about 40°, about 41°, about 42°, about 42°, about 43°, about 44°, about 45°, about 46°, about 47°, about 48°, about 49°, about 50°, about 51°, about 52°, about 53°, about 54°, about 55°, about 56°, about 57°, about 58°, about 59°, about 60°, about 61°, about 62°, about 63°, about 64°, or even about 65°.

Likewise, the second pair of opposed cutting edges may have a maximum width extending between the opposed cutting edges as measured at or near the junction of about 0.5mm, about 0.6mm, about 0.7mm, about 0.8mm, about 0.9mm, about 1.0mm, about 1.1mm, about 1.2mm, about 1.3mm, about 1.4mm, about 1.5mm, about 1.6mm, about 1.7mm, about 1.8mm, about 1.9mm, or even about 2.0mm, typically ranging between about 0.5mm and about 1.2mm, preferably about 1.0mm.

In embodiments of the blade according to the first aspect, the second blade portion may have a second angle of between about 10° and about 15°, preferably 12.5°.

In embodiments of the blade according to the second aspect, the second blade portion may have a second angle of between about 30° and about 60°.

Advantageously, the second blade portion by having a smaller blade width relative to the first blade portion is able to accurately and repeatedly form a secondary corneal incision or side port incision of precise width when the second blade portion is advanced into the eye in a direction parallel with the central axis, typically up to substantially parallel side edge portions.

Conversely, the greater blade width of the first blade portion enables the primary incision to be accurately and repeatedly formed when the first blade portion is advanced into the eye in a direction parallel with the central axis, typically up to the apex.

The second blade portion may be of any suitable length, as defined between the blade tip and the junction. Preferably, the second blade portion may be not too long so as to protect against possible over extension into the anterior chamber and/or causing possible injury to the endothelium when forming a side portion incision. For example, the second blade portion may have a length of about 0.6mm, about 0.7mm, about 0.8mm, about 0.9mm, about 1.0mm, about 1.1mm, or about 1.2mm or more. Typically, the second blade portion may have a length of between about 0.7mm and about 1.2mm, preferably between about 0.7mm and 1.1mm, more preferably between about 0.7mm and 1.0mm.

In preferred embodiments, the second blade portion may have a length directly proportion to a width of the second blade portion. For example, the second blade portion may have a length equal to or less than a multiple of 1.2 times a maximum width of the second blade portion, preferably a multiple of between 0.7 and 1.1 times the maximum width, more preferably a multiple of between 0.7 and 1.0 times the maximum width.

In more preferred embodiments, the second blade portion may have a length of about 1.0mm or 1.0 times the maximum width of the second blade portion.

Advantageously, by having a length of between about 0.7mm and about 1.2mm, the risk of injury to the endothelium or of over extension of the second blade portion into the anterior chamber when forming a side port incision is greatly reduced. Further, the substantially parallel side edge portions of the second blade portion provide clear visual feedback to a surgeon when advancing the second blade portion into the anterior chamber of the eye and forming the side portion incision.

In some embodiments, the blade may include one or more markings or etchings to assist an ophthalmologist in visualising at least when the first blade portion has been advanced into the eye, preferably also when the second blade portion has been advanced into the eye.

The one or markings may be of any suitable form and may be arranged in any suitable location on the blade.

For example, in some such embodiments, the markings may include one or more symbols or indicia applied or etched at least partially along the upper surface.

In some such embodiments, the markings may include a reflective or fluorescent dye to assist the ophthalmologist in visualising the markings during a surgical procedure.

In preferred embodiments, the blade may include a first marking extending laterally across an upper surface of the blade in a direction substantially perpendicular to the central axis to indicate a suitable depth of insertion for forming a primary incision, and a second marking extending laterally across an upper surface of the blade in a direction substantially perpendicular to the central axis for forming a side port incision.

In some such embodiments, the first marking may be located at the apex and the second marking may be located at the junction.

In other such embodiments, the first marking may be located between about 2.0mm and about 4.0mm from the blade tip and the second marking may be located between about 0.7mm and 1.0mm from the blade tip.

An ophthalmic surgical instrument for delivery or removal of fluid into or from an eye can be provided.

The fluid may be viscoelastic material as used in cataract extraction surgery. The fluid may be aqueous humour as drained from an eye to lower intraocular pressure inside the eye in patients with glaucoma, i.e., a glaucoma bleb needling procedure. The fluid may be a biopsy sample of body fluid obtained from an eye.

The instrument may be of any size, shape and construction and formed from any material or materials suitably adapted for making an incision in an eye for delivery and/or removal of fluid by a source, such as, e.g., a syringe. Preferably, the incision may be a clear corneal incision for delivery and/or removal of fluid from an anterior chamber of the eye.

Generally, the shaft and the blade portion of the instrument may be integrally formed and typically formed from any material or materials capable of forming a sharp cutting edge. Usually, the instrument may be formed from metal and/or plastic material or materials, preferably stainless steel, titanium or aluminium.

The hollow shaft may be of any suitable size, shape and length for connecting the blade portion to the source of fluid and providing a conduit therebetween.

Generally, the shaft includes a pair of opposed ends and extends longitudinally between the opposed ends. The opposed ends may include a proximal end connectable to the source or a cannula and an opposed distal end from which the blade portion extends. The opposed ends may preferably be open ends to enable the fluid to be delivered and removed from an eye via the distal end.

The shaft may preferably be of tubular construction and have a substantially circular cross-section.

The shaft may extend in a linear direction between the source and the blade portion. The shaft may include one or more bends. For example, the shaft may include a distal portion and a proximal portion angled relative to one another. For example, it is envisaged that the distal portion may be extend relative to the proximal portion at an angle ranging between about 120° and about 150°, preferably an angle ranging between about 130° and about 140°, more preferably an angle of about 135°.

The proximal end may be connectable to the source or cannula in any suitable way to provide fluid communication between the source and the distal end of the shaft and to ensure that the parts do not inadvertently disconnect during a surgical procedure. For example, the proximal end may be directly or indirectly connectable to the source or the cannula. Preferably, the proximal end may be detachably connectable.

The proximal end of the shaft may be connected to the source or the cannula by one or more fasteners, such as, e.g., one or more mechanical fasteners and/or one or more chemical fasteners, preferably the latter.

The chemical fasteners may include a wet adhesive, a dry adhesive and/or double-sided adhesive tape that may extend between the proximal end of the shaft or part thereof and the source or cannula or part thereof.

The proximal end of the shaft may be connected to the source or the cannula by a connecting mechanism or part of a connecting mechanism. The connecting mechanism may include a first part associated with the proximal end of the shaft and a second part connectable to the first part and associated with the source or the cannula.

The parts of the connecting mechanism may respectively include mateable male and female portions that couple together, including threaded connections, interference fit connections or bayonet-type connections, for example.

For example, the first part of the connecting mechanism associated with the proximal end of the shaft may include a male formation configured to be inserted into or coupled with a female formation of the second part of the connecting mechanism associated with the source or the cannula. Conversely, the first part of the connecting mechanism associated with the proximal end of the shaft may include a female formation configured to receive or be coupled with a male formation of the second part of the connecting mechanism associated with the source or the cannula.

The proximal end of the shaft and the source or cannula may be connectable by way of a Luer taper system. For example, the proximal end of the shaft may include a male or female Luer-Lok^{™} connection fitting configured to be respectively mateable with the other of a female or male Luer-Lok^{™} connection fitting associated with the cannula or source. For example, the proximal end of the shaft may include a male Luer-Lok^{™} connection mateable with a female Luer-Lok^{™} connection associated with a syringe.

The proximal end of the shaft and the source or cannula may be connectable by way of a friction fit. For example, the proximal end of the shaft may include a slip tip fitting configured to frictionally fit over an end of a cannula or a syringe tip.

The shaft may be of any suitable length. For example, the shaft may be about 5mm, about 6mm, about 7mm, about 8mm, about 9mm, about 10mm, about 11mm, about 12mm, about 13mm, about 14mm, about 15mm, about 16mm, about 17mm, about 18mm, about 19mm or even about 20mm in length. Typically, the shaft may have a length of between about 6mm and about 12mm.

The shaft may partially taper or flatten at or near the distal end from which the blade portion extends, preferably partially flatten. For example, the distal end of the shaft may have an oval or even triangular cross-sectional shape. Advantageously, this may provide a more streamline shape and facilitate insertion of the distal end of the shaft into an incision formed by the corresponding blade portion.

As indicated, the blade portion may extend from the shaft along a central axis to a blade tip and include at least one side edge portion with a cutting edge thereon. The cutting edge being oriented at a first angle relative to the central axis and extending to the blade tip, preferably such that the cutting edge faces, or at least partially faces, towards the blade tip to facilitate incision formation when the blade portion is advanced into the eye in a direction parallel with the central axis.

Generally, the blade portion may extend from the distal end of the shaft along the central axis to the blade tip, preferably a pointed blade tip.

Typically, the blade portion may be in the form of a plate. The portion may usually be substantially flat or planar, although it is envisaged that parts or portions of the blade portion may at least partially extend around a circumference of the distal end of the shaft.

The blade portion may be of any suitable size, shape and construction. For example, the blade portion may have a substantially triangular, diamond or kite profile shape.

The blade portion may have two **opposed** surfaces, including an inner surface and an opposed outer surface. The opposed surfaces may extend substantially parallel to one another and be interconnected by opposing edges, including opposed side edges, a proximal end edge from which the blade portion extends from the distal end of the shaft and an opposed distal end edge defining the blade tip.

In preferred embodiments, the blade portion may include a pair of opposed side edge portions that extend about the central axis from at or near the distal end of the shaft to the blade tip. The side edge portions may correspond to the side edges. The side edge portions may be symmetrical or asymmetrical.

In some embodiments, the side edge portions may extend in a linear direction from the distal end of the shaft to the blade tip. In other embodiments, at least one side edge portion may flare and taper respectively away and at least partially towards the central axis as it extends between the proximal end and the distal end of the blade portion. Again, the side edge portions may flare and taper in linear or curvilinear directions, or a combination thereof.

Typically, a proximal end facing side edge portion may have a rounded or non-cutting edge so as not to modify an incision, i.e., enlarge the incision or cause superficial cuts, as the blade portion is retracted.

In some embodiments, a single cutting edge may be defined on at least a portion of one of the side edge portions, typically a distal-end facing side edge portion. In other embodiments, a cutting edge may be defined on at least a portion of each side edge portion, again typically distal-end facing side edge portions.

In some embodiments, the blade portion may include a first side edge portion that extends linearly between the proximal end and the distal end and a second side edge portion that flares out at or near the proximal end to define a proximal-end facing side edge portion, a widest part or apex of the blade portion and a distal-end facing side edge portion that gently tapers toward the blade tip, preferably in a linear direction. The cutting edge in such embodiments may preferably be defined on the distal-end facing side edge portion.

In other embodiments, the blade portion may include a pair of opposed side edge portions that each flare out and away from the central axis at or near the proximal end to define proximal-end facing side edge portions, a widest part or apex of the blade portion, and distal-end facing side edge portions that gently taper or converge at least partially towards the central axis to the blade tip, preferably in a linear direction. In such embodiments, cutting edges may preferably be defined on both distal-end facing side edge portions.

In some embodiments, the distal end of the shaft and the proximal end of the blade portion may at least partially overlap, preferably such that at least one said proximal-end facing side edge portion overlaps the distal end of the shaft. Advantageously, this enables the distal end of the shaft to be readily inserted into the incision as the blade portion is advanced into the eye in a direction parallel with the central axis.

In other embodiments, the proximal end of the blade portion may extend at least partially about a circumference of the distal end of the shaft. Advantageously, this also enables the distal end of the shaft to be readily inserted into the incision as the blade portion is advanced into the eye in a direction parallel with the central axis.

As indicated, the cutting edge of the blade portion is oriented at an angle relative to the central axis, preferably such that the cutting edge faces, or at least partially faces towards the blade tip to facilitate incision formation when the blade portion is advanced into the eye in a direction parallel with the central axis.

The angle may preferably be an acute angle. For example, the angle may be about 5°, about 6°, about 7°, about 8°, about 9°, about 10°, about 11°, about 12°, about 13°, about 14°, about 15°, about 16°, about 17°, about 18°, about 19°, or even about 20°, preferably about 12.5°.

Likewise, the blade portion may have a maximum width, as measured at the widest point between the opposed sides or the apex in some embodiments, of about 0.5mm, about 0.6mm, about 0.7mm, about 0.8mm, about 0.9mm, about 1.0mm, about 1.1mm, about 1.2mm, about 1.3mm, about 1.4mm, about 1.5mm, about 1.6mm, about 1.7mm, about 1.8mm, 1.9mm, or even about 2.0mm, typically ranging between about 0.5mm and about 1.0mm, preferably about 0.6mm.

A method of forming a primary incision and a side port incision during cataract surgery is described. Said method not forming part of this invention but providing background information, said method including:
providing the multi-purpose ophthalmic blade of the first or second aspects;
forming the side port incision in an eye by at least partially advancing the blade at a first location into an anterior chamber of the eye to a junction between the first and second blade portions; and
forming the primary incision in the eye by at least partially advancing the blade at a second location into the anterior chamber of the eye to an end of the opposed first cutting edges of the first blade portion.

The method may include one or more features or characteristics of the multi-purpose blade as hereinbefore described.

Preferably, the first and second locations may be located at about 60-90° relative to one another about the corneoscleral junction of the eye.

Preferably, the forming the primary incision may include advancing the blade into the anterior chamber of the eye to the apex of the blade, typically to create a 2.2 mm (width) by 4mm (length) incision.

The forming the primary incision may be performed before the forming the side port incision.

A method of delivering or removing fluid into or out of an eye is described. Said method does not form part of this invention but serves explanatory reasons only, said method including:
providing the ophthalmic surgical instrument of the third or fourth aspects; and
forming a side port incision in the eye by at least partially advancing the blade portion into the eye until an outer end of the shaft is in fluid communication with the incision for delivery or removal of the fluid.

The method may include one or more features or characteristics of the ophthalmic surgical instrument as hereinbefore described.

The forming may include advancing the blade portion into the eye until the blade is entirely inserted into an anterior chamber of the eye, preferably forming a clear corneal incision.

The forming may include advancing the blade portion up to the apex into the eye, preferably through a clear cornea portion of the eye.

The method may further include delivering or removing the fluid by way of a syringe operatively associated with the ophthalmic surgical instrument. The delivering or removal of fluid may be by way of a pump operatively associated with the ophthalmic surgical instrument.

Any of the features described herein can be combined in any combination with any one or more of the other features described herein within the scope of the invention.

The reference to any prior art in this specification is not, and should not be taken as an acknowledgement or any form of suggestion that the prior art forms part of the common general knowledge.

The invention is defined by the appended claims.

### BRIEF DESCRIPTION OF DRAWINGS

Preferred features, embodiments and variations of the invention may be discerned from the following Detailed Description which provides sufficient information for those skilled in the art to perform the invention. The Detailed Description is not to be regarded as limiting the scope of the preceding Summary of Invention in any way. The Detailed Description will make reference to a number of drawings as follows:
Figure 1 is an upper perspective view of a multi-purpose ophthalmic blade.
Figure 2 is a side view of the multi-purpose ophthalmic blade as shown in Figure 1;
Figure 3 is an upper perspective view of a multi-purpose ophthalmic blade.
Figure 4 is a plan view of a multi-purpose ophthalmic blade according to an embodiment of the present invention;
Figure 5 is a plan view of a multi-purpose ophthalmic blade according to another embodiment of the present invention;
Figure 6 is a plan view of a multi-purpose ophthalmic blade according to another embodiment of the present invention;
Figure 7 is an upper perspective view of a surgical instrument.
Figure 8 is another upper perspective view of the surgical instrument as shown in Figure 7 operatively associated with a cannula;
Figure 9 is an upper perspective view of a surgical instrument.
Figure 10 is another upper perspective view of the surgical instrument as shown in Figure 9 operatively associated with a cannula;
Figure 11 is an upper perspective view of a surgical instrument.
Figure 12 is another upper perspective view of the surgical instrument as shown in Figure 11 operatively associated with a cannula;
Figure 13 is an upper perspective view of a surgical instrument; and
Figure 14 is another upper perspective view of the surgical instrument as shown in Figure 13 operatively associated with a cannula.

### DETAILED DESCRIPTION

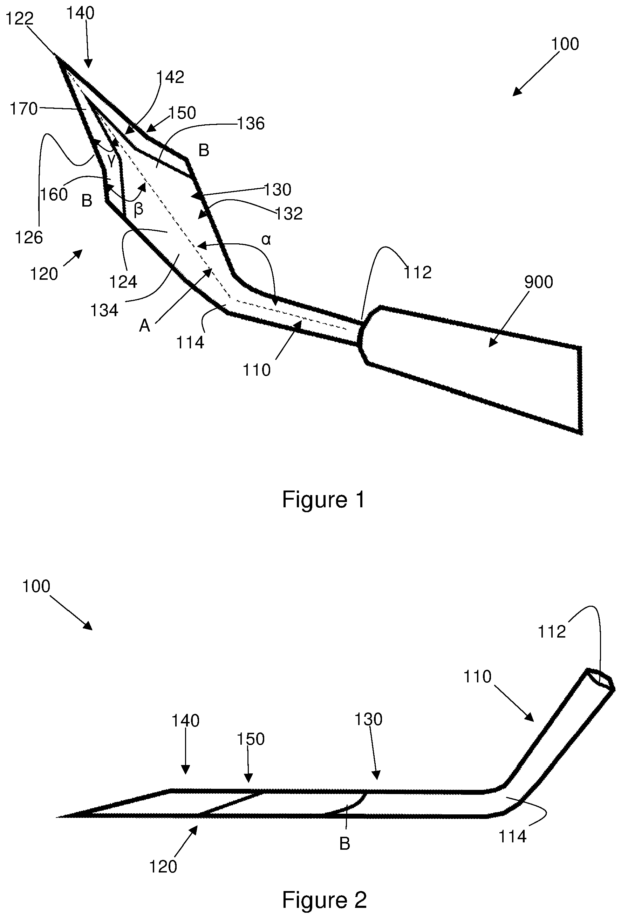

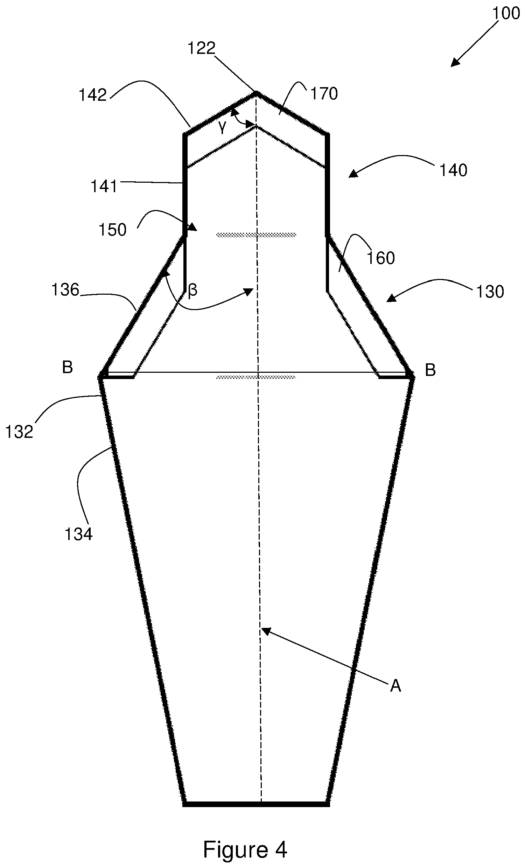

Figures 1 to 6 show examples of a multi-purpose ophthalmic blade (100) wherein Figures 4 to 6 show embodiments according to the present invention for use in cataract extraction surgery.

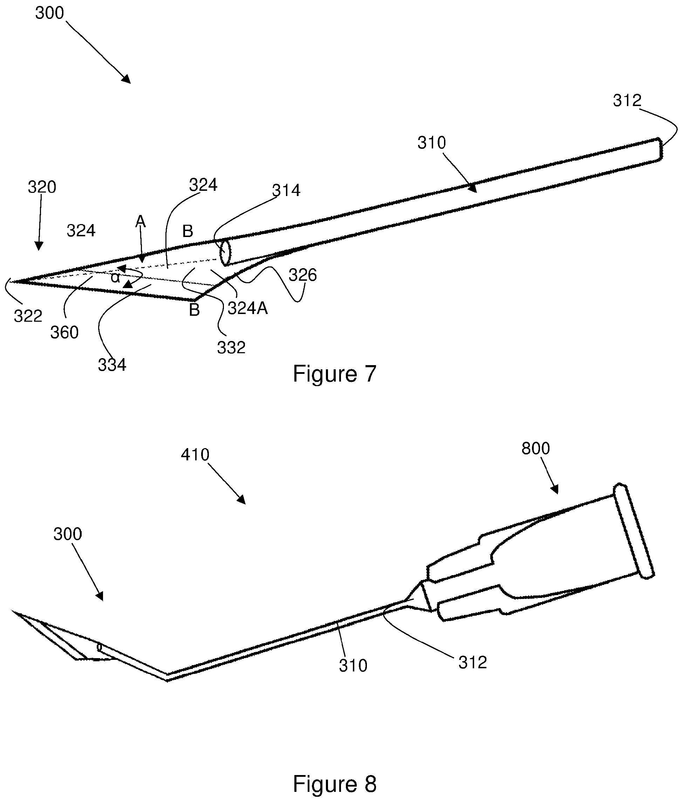

Figures 7 to 14 show an ophthalmic surgical instrument (300) not being part of the present invention.

Referring to Figures 1 and 2, these figures show a multi-purpose ophthalmic blade (100) not being part of the present invention. The multi-purpose ophthalmic blade (100) has a first blade portion (130) and a second blade portion (140) for respectively forming a primary incision and a side port incision during cataract extraction surgery.

The blade (100) is integrally formed from stainless steel.

The blade (100) includes a shaft (110) connectable to a handle (900; shown only in Figure 1) and a blade body (120) defining the first blade portion (130) and the second blade portion (140).

The shaft (110) extends longitudinally in a linear direction between a pair of opposed ends, including a proximal end (112) connectable to the handle (900) and an opposed distal end (114) from which the blade body (120) and portions (130, 140) extend.

The shaft (110) is of solid construction and has a substantially circular cross-section that tapers or flattens at or near the distal end (114).

The handle (900; shown only in Figure 1) has an elongate shape suitable for being held and manipulated by a hand of an ophthalmologist. The handle (900; shown only in Figure 1) is formed from plastic material or materials and is overmoulded over the proximal end (112) of the shaft (110).

The shaft (110) extends a length of about 9mm between the handle (900) and the blade body (120).

As indicated, the shaft (110) tapers or flattens at or near the distal end (114) from which the blade body (120) extends at an angle (α) of about 135°relative to the shaft (110).

Referring to Figure 1, the blade body (120) extends from the distal end (114) of the shaft along a central axis (A) to a pointed blade tip (122). The body (120) is in the form of a plate having a substantially diamond or kite shape.

The body (120) has two opposed surfaces, including an upper surface (124) that faces outwards of an eye during surgery and an opposed lower surface (126). The opposed surfaces (124, 126) extend substantially parallel to one another and are interconnected by opposing edges, including opposed side edges, a proximal end edge from which the body (120) extends from the shaft (110), and an opposed distal end edge defining the blade tip (122) of the second blade portion (140).

The body (120) defines the first blade portion (130) at or near a proximal end of the body, the second blade portion (140) at or near the distal end of the body (120) and a junction (150) between the first and second blade portions (130, 140).

The first blade portion (130) and the second blade portion (140) extend from the shaft (110) along the central axis (A). The first blade portion (130) includes a pair of opposed side edge portions (132) that extend symmetrically about the central axis (A).

The side edge portions (132) flare gently flare outwards and away from the central axis (A) as they extend along from the proximal end at least partially towards the junction (150) to define a widest part of the blade or apex (B:B). The side edge portions (132) then gently taper at least partially towards the central axis (A) as they extend from the apex (B:B) towards the junction (150).

The apex (B:B) defines proximal end facing side edge portions (134) extending between the proximal end and the apex (B:B) and distal-end facing side edge portions (136) extending between the apex (B:B) and the junction (150).

A first pair of cutting edges (160) are defined on the distal-end facing side edge portions (136).

The proximal end facing side edge portions (134) have rounded or non-cutting edges so as not to modify the incision, i.e., enlarge the incision or cause superficial cuts, as the blade (100) is retracted.

The second blade portion (140) extends distally forward from the first blade portion (130) the central axis (A) to the blade tip (122). Like the first blade portion (130), the second blade portion (140) includes a pair of opposed side edge portions (142) that extend symmetrically about the central axis (A) and converge to the blade tip (122). The second pair of cutting edges (170) are defined on the side edge portions (142).

The first pair of cutting edges (160) extend continuously into the second pair of cutting edges (170) save that the pairs (160, 170) are oriented at different angles relative to the central axis (A).

Each pair of cutting edges (160, 170) is oriented at a blade angle relative to the central axis (A) such that they at least partially face towards the blade tip (122) to facilitate incision formation when the blade (100) is advanced into an eye in a direction parallel with the central axis (A).

The first pair of cutting edges (160) each have a blade angle (β) of about 22° relative to the central axis (A) that is greater than the blade angle (γ) of 12.5° at which the second pair of cutting edges (170) are each oriented relative to the central axis (A).

The first pair of cutting edges (160) have a maximum width extending between the opposed cutting edges, as measured at or near the apex (B:B), of about 2.4mm.

In contrast, the second pair of opposed cutting edges (170) have a maximum width extending between the opposed cutting edges (170) as measured at or near the junction (150) of about 0.6mm.

Referring to both Figures 1 and 2, in use, an ophthalmologist can form the side port incision by advancing the blade (100) up to the junction (150) into the cornea at a first location of the eye.

The ophthalmologist can then form the primary incision with the same blade (100) by advancing the blade (100) up to the apex (B:B) into the cornea at a second location of the eye in a direction substantially parallel with the central axis (A).

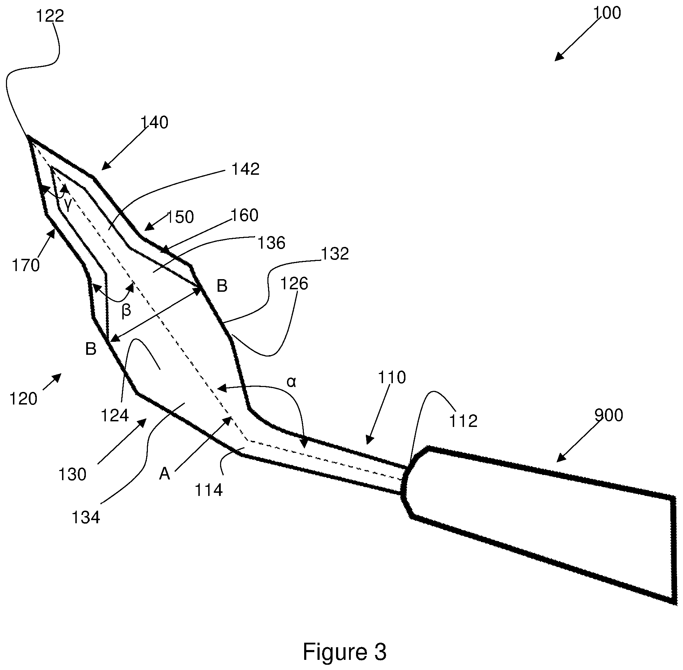

Figure 3 shows another multi-purpose ophthalmic blade (100) not being part of the present invention. For convenience, features that are similar or correspond to features of the first ophthalmic blade will again be referenced with the same reference numerals.

The multi-purpose ophthalmic blade (100) has a first blade portion (130) and a second blade portion (140) for respectively forming a primary incision and a side port incision.

The blade (100) includes a shaft (110) connectable to a handle (900) and a blade body (120) defining the first blade portion (130) and the second blade portion (140).

The shaft (110) and the handle (900) are the same as described above.

The blade body (120) extends from the distal end (114) of the shaft along a central axis (A) to a pointed blade tip (122). The body (120) is in the form of a plate.

The body (120) has two opposed surfaces, including an upper surface (124) that faces outwards of an eye during surgery and an opposed lower surface (126). The opposed surfaces (124, 126) extend substantially parallel to one another and are interconnected by opposing edges, including opposed side edges, a proximal end edge from which the body (120) extends from the shaft (110), and an opposed distal end edge defining the blade tip (122) of the second blade portion (140).

The body (120) defines the first blade portion (130) at or near a proximal end of the body, the second blade portion (140) at or near the distal end of the body (120) and a junction (150) between the first and second blade portions (130, 140).

The first blade portion (130) and the second blade portion (140) extend from the shaft (110) along the central axis (A). The first blade portion (130) includes a pair of opposed side edge portions (132) that extend symmetrically about the central axis (A).

The side edge portions (132) flare gently flare outwards and away from the central axis (A) as they extend along from the proximal end at least partially towards the junction (150) to define a widest part of the blade or apex (B:B). The side edge portions (132) then gently taper at least partially towards the central axis (A) as they extend from the apex (B:B) towards the junction (150).

The apex (B:B) defines proximal end facing side edge portions (134) extending between the proximal end and the apex (B:B) and distal-end facing side edge portions (136) extending between the apex (B:B) and the junction (150).

A first pair of cutting edges (160) are defined on the distal-end facing side edge portions (136).

The proximal end facing side edge portions (134) have rounded or non-cutting edges so as not to modify the incision, i.e., enlarge the incision or cause superficial cuts, as the blade (100) is retracted.

The second blade portion (140) extends distally forward from the first blade portion (130) the central axis (A) to the blade tip (122). Like the first blade portion (130), the second blade portion (140) includes a pair of opposed side edge portions (142) that extend symmetrically about the central axis (A) and converge to the blade tip (122). The second pair of cutting edges (170) are defined on the side edge portions (142).

The first pair of cutting edges (160) extend continuously into the second pair of cutting edges (170) save that the pairs (160, 170) are oriented at different angles relative to the central axis (A).

The first pair of cutting edges (160) each have a blade angle (β) of about 22° relative to the central axis (A) that is greater than the blade angle (γ) of 12.5° at which a distal portion of the second pair of cutting edges (170) are each oriented relative to the central axis (A) and converge to the blade tip (122).

The first pair of cutting edges (160) have a maximum width extending between the opposed cutting edges, as measured at or near the apex (B:B), of about 2.4mm.

In contrast, the second pair of opposed cutting edges (170) have a maximum width extending between the opposed cutting edges (170) as measured at or near the junction (150) of about 0.6mm.

Further, the second pair of opposed cutting edges (170) includes the distal portion that converges to the blade tip (122) and a proximal portion in which the opposed cutting edges (170) extend substantially parallel to one another from the distal portion to the junction (150).

Figure 4 shows the multi-purpose ophthalmic blade (100) according to an embodiment of the present invention. For convenience, features that are similar or correspond to features of the other ophthalmic blades will again be referenced with the same reference numerals.

The multi-purpose ophthalmic blade (100) has a first blade portion (130) and a second blade portion (140) for respectively forming a primary incision and a side port incision during cataract extraction surgery.

The first blade portion (130) includes a pair of opposed side edge portions (132) that extend symmetrically about central axis (A).

The side edge portions (132) flare gently flare outwards and away from the central axis (A) as they extend along from the proximal end at least partially towards the junction (150) to define a widest part of the blade or apex (B:B). The side edge portions (132) then gently taper at least partially towards the central axis (A) as they extend from the apex (B:B) towards the junction (150).

The apex (B:B) defines proximal end facing side edge portions (134) extending between the proximal end and the apex (B:B) and distal-end facing side edge portions (136) extending between the apex (B:B) and the junction (150).

A first pair of cutting edges (160) are defined on the distal-end facing side edge portions (136).

The proximal end facing side edge portions (134) have rounded or non-cutting edges so as not to modify the incision, i.e., enlarge the incision or cause superficial cuts, as the blade (100) is retracted.

The second blade portion (140) extends distally forward from the first blade portion (130) along the central axis (A) to the blade tip (122). The second blade portion (140) includes a pair of opposed parallel side edge portions (141) without cutting edges defined thereon and a pair of converging side edge portions (142) extending distally forward from the parallel side edge portions (141). The converging side edge portions (142) converge to the blade tip (122). The second pair of cutting edges (170) are defined on the converging side edge portions (142).

The first pair of cutting edges (160) and the second pair of cutting edges (170) are oriented at different angles relative to the central axis (A).

The first pair of cutting edges (160) each have a blade angle (β) of about 33° relative to the central axis (A). The second pair of cutting edges (170) each have a blade angle (γ) of about 60° relative to the central axis (A).

The first pair of cutting edges (160) have a maximum width extending between the opposed cutting edges, as measured at or near the apex (B:B), of about 2.2mm.

In contrast, the second pair of opposed cutting edges (170) have a maximum width extending between the opposed cutting edges (170) as measured at or near the junction (150) or between opposed parallel side edge portions (141) of about 1.0mm.

The second blade portion (140) has a length, as measured between the blade tip (122) and the junction (150) of about 1.0mm or a multiple of 1.0 times the maximum width of the second blade portion (140).

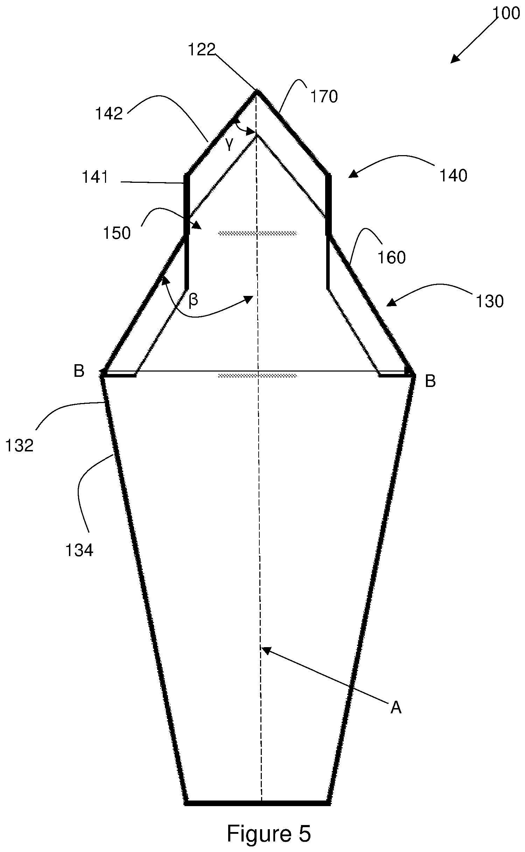

Figure 5 shows the multi-purpose ophthalmic blade (100) according to another embodiment of the present invention. For convenience, features that are similar or correspond to features of the other ophthalmic blades will again be referenced with the same reference numerals.

The blade (100) according to the fourth embodiment is substantially the same as the blade (100) shown in the third embodiment save that the second blade portion (140) has a blade angle (γ) of about 42° relative to the central axis (A).

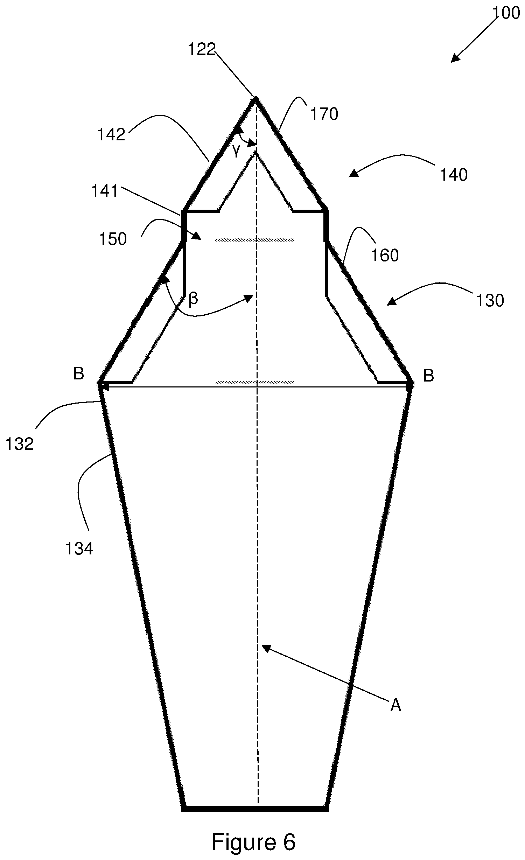

Figure 6 shows the multi-purpose ophthalmic blade (100) according to a fifth embodiment of the present invention. For convenience, features that are similar or correspond to features of the other ophthalmic blades will again be referenced with the same reference numerals.

The blade (100) according to the fifth embodiment is substantially the same as the blades (100) shown in the third and fourth embodiments save that the second blade portion (140) has a blade angle (γ) of about 33° relative to the central axis (A).

Figure 7 shows a surgical instrument (300) not being part of the present invention for delivery and removal of viscoelastic fluid into or from an eye in a direction substantially parallel with the central axis (A).

The instrument (300) includes a hollow shaft (310) connectable to a syringe or cannula in fluid communication with a source of the viscoelastic fluid and a blade portion (320) adapted for making a sclerocorneal tunnel or side portion incision in the eye for the delivery or removal of the viscoelastic fluid.

The shaft (310) and the blade portion (320) of the instrument (300) are integrally formed from stainless steel.

The hollow shaft (310) is sized and shaped for connecting the blade portion (320) to the source of viscoelastic fluid and providing a conduit therebetween.

The shaft (310) includes a pair of opposed ends and extends longitudinally therebetween. The opposed ends include a proximal end (312) connectable to the source or cannula and an opposed distal end (314) from which the blade portion (320) extends. The opposed ends (312, 314) are open ends to enable the viscoelastic fluid to be delivered and removed from an eye via the distal end (314).

The shaft (310) is of tubular construction and has a substantially circular cross-section.

The shaft (310) has a length of between about 6mm and about 12mm and a diameter of about 0.6-0.8mm.

As indicated, the blade portion (320) extends from the shaft (310) along a central axis (A) to a blade tip (322) and includes a pair of asymmetrical side edge portions (324) with a cutting edge (360) at least partially defined on one side edge portion (324A).

The cutting edge (360) is oriented at a blade angle (α) of about 12.5° relative to the central axis (A) and extends to the blade tip (322) such that the cutting edge (360) at least partially faces towards the blade tip (322) to facilitate incision formation when the blade portion (320) is advanced into the eye in a direction parallel with the central axis (A).

The blade portion (320) is in the form of a plate having a substantially triangular, diamond or kite profile shape.

The blade portion (320) has two opposed surfaces, including an inner surface (324) and an opposed outer surface (326). The opposed surfaces (324, 326) extend substantially parallel to one another are interconnected by opposing edges, including opposed side edges, a proximal end edge from which the blade portion (320) extends from the distal end (314) of the shaft (310) and an opposed distal end edge defining the blade tip (322).

In the example shown, the side edge portion (324A) upon which the cutting edge (360) is defined flares out at or near the proximal end to define a proximal-end facing side edge portion (332), a widest part or apex (B:B) of the blade portion (320) and a distal-end facing side edge portion (334) that gently tapers toward the blade tip (322) in a linear direction. The cutting edge (360) is defined on the distal-end facing side edge portion (334).

The distal end (314) of the shaft (310) and the proximal-end facing side edge portion partially overlap. Advantageously, this enables the distal end (314) of the shaft (310) to be readily inserted into the incision as the blade portion (320) is advanced into the eye in a direction parallel with the central axis (A).

The blade portion (320) may have a maximum width, as measured at the apex (B:B), of about 0.8mm.

The proximal-end facing side edge portion (332) has a rounded or non-cutting edge so as not to modify an incision, i.e., enlarge the incision or cause superficial cuts, as the blade portion (320) is retracted.

In the example shown, the proximal end (312) of the shaft (310) may be connectable to a cannula by way of a friction fit.

Referring to Figure 8, the instrument (300) shown in Figure 7 is shown in the form of a cannula (410) having a male Luer-Lok^{™} connection fitting (800) for connecting to a syringe containing the viscoelastic fluid, for example.

The male Luer-Lok^{™} connection fitting (800) is overmoulded over the proximal end (312) of the shaft (310). Further, the shaft (310) including distal portion angled relative to a proximal portion at an angle of about 135°.

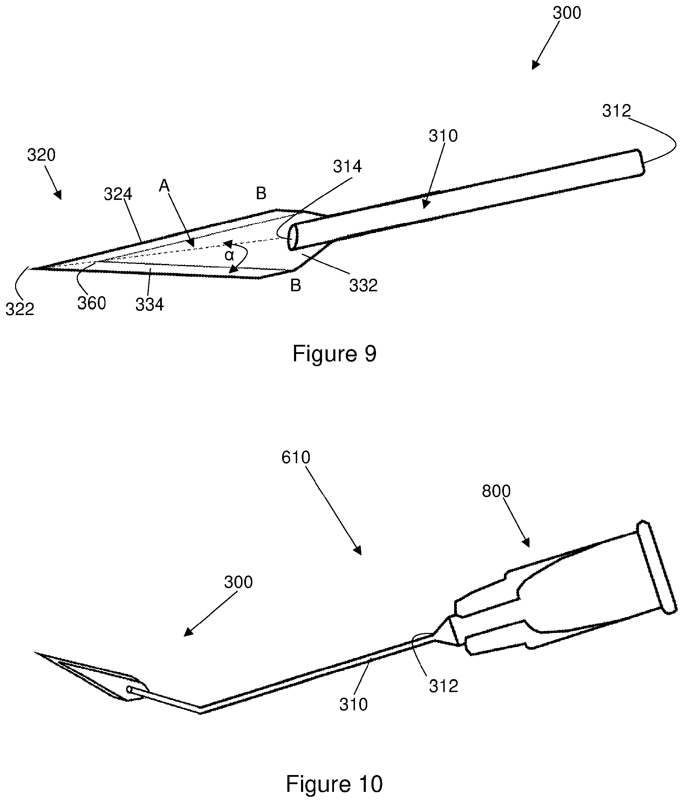

Figure 9 shows a surgical instrument (300) for delivery and removal of viscoelastic fluid into or from an eye. For convenience, features that are similar or correspond to features of the first surgical instrument will again be referenced with the same reference numerals.

The surgical instrument (300) includes a shaft (310) as previously described.

However, in contrast to the first example, the blade portion (320), which extends from the shaft (310) along a central axis (A) to a blade tip (322), includes a pair of symmetrical side edge portions (324) with cutting edges (360) at least partially defined on both side edge portions (324).

Both side edge portions (324) flare out at or near the proximal end to define proximal-end facing side edge portions (332), a widest part or apex (B:B) of the blade portion (320) and distal-end facing side edge portions (334) that gently tapers toward the blade tip (322) in a linear direction. The cutting edges (360) is defined on the distal-end facing side edge portions (334).

The cutting edges (360) are each oriented at a blade angle (α) of about 12.5° relative to the central axis (A) and extend to the blade tip (322) such that the cutting edges (360) at least partially faces towards the blade tip (322) to facilitate incision formation when the blade portion (320) is advanced into the eye in a direction parallel with the central axis (A).

The blade portion (320) has a maximum width, as measured at the apex (B:B), of about 0.8mm.

The proximal-end facing side edge portions (332) each have a rounded or non-cutting edge so as not to modify an incision, i.e., enlarge the incision or cause superficial cuts, as the blade portion (320) is retracted.

The proximal end (312) of the shaft (310) is connectable to a cannula by way of a friction fit.

Referring to Figure 7, the instrument (300) shown in Figure 6 is shown in the form of a cannula (610) having a male Luer-Lok^{™} connection fitting (800) for connecting to a syringe containing the viscoelastic fluid, for example.

The male Luer-Lok^{™} connection fitting (800) is overmoulded over the proximal end (312) of the shaft (310). Further, the shaft (310) including distal portion angled relative to a proximal portion at an angle of about 135°.

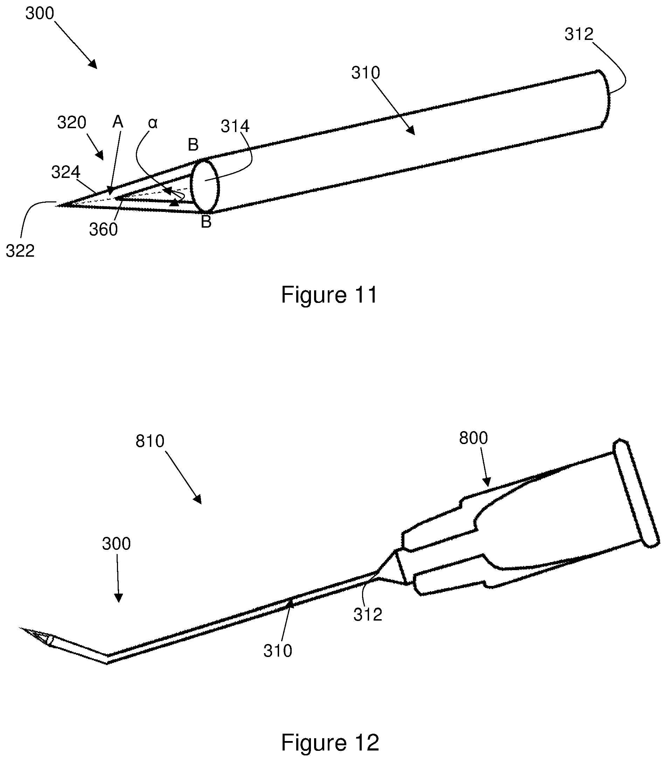

Figure 11 shows a surgical instrument (300) not being part of the present invention for delivery and removal of viscoelastic fluid into or from an eye. For convenience, features that are similar or correspond to features of the previous examples will again be referenced with the same reference numerals.

The surgical instrument (300) includes a shaft (310) as previously described.

The blade portion (320) extends from the shaft (310) along a central axis (A) to a blade tip (322), and includes a pair of symmetrical side edge portions (324) with cutting edges (360) defined on both side edge portions (324).

The cutting edges (360) are each oriented at a blade angle (α) of about 12.5° relative to the central axis (A) and extend to the blade tip (322) such that the cutting edges (360) at least partially faces towards the blade tip (322) to facilitate incision formation when the blade portion (320) is advanced into the eye in a direction parallel with the central axis (A).

In contrast to the previous examples , the proximal end of the blade portion (320) extends at least partially about a circumference of the distal end (312) of the shaft (310). Advantageously, this enables the distal end (312) of the shaft (310) to be readily inserted into the incision as the blade portion (320) is advanced into the eye in a direction parallel with the central axis (A).

Further, both side edge portions (324) gently taper or converge toward the blade tip (322) in a linear direction from the proximal end. Accordingly, the proximal end is the widest part or apex (B:B) of the blade portion (320) and has a maximum width of about 0.6mm.

In the example shown, the proximal end (312) of the shaft (310) is connectable to a cannula by way of a friction fit.

Referring to Figure 12, the instrument (300) shown in Figure 11 is shown in the form of a cannula (810) having a male Luer-Lok^{™} connection fitting (800) for connecting to a syringe containing the viscoelastic fluid, for example.

The male Luer-Lok^{™} connection fitting (800) is overmoulded over the proximal end (312) of the shaft (310). Further, the shaft (310) including distal portion angled relative to a proximal portion at an angle of about 135°.

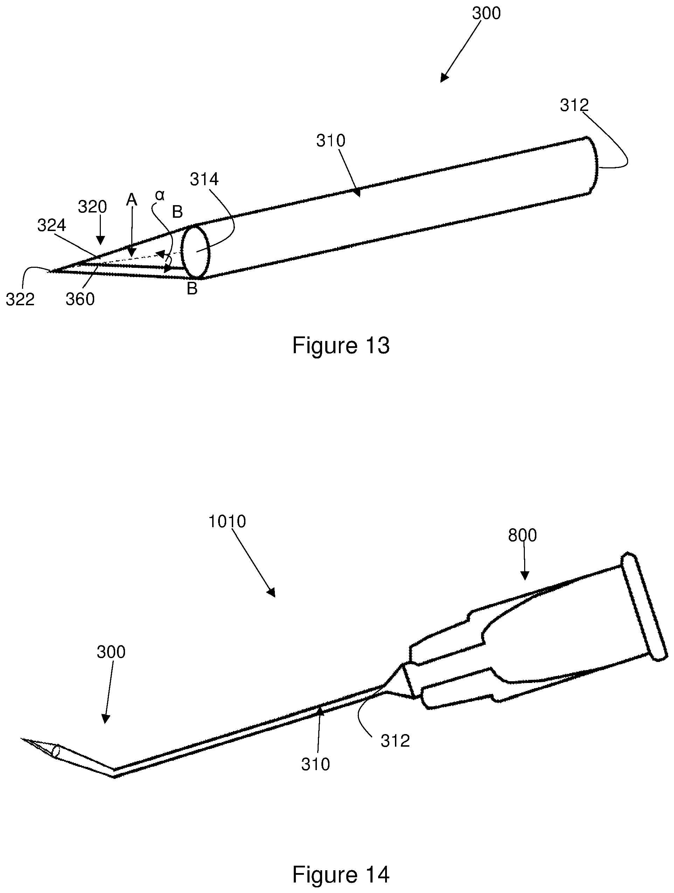

Figure 13 shows a surgical instrument (300) not being part of the present invention for delivery and removal of viscoelastic fluid into or from an eye. For convenience, features that are similar or correspond to features of the previous instruments will again be referenced with the same reference numerals.

The surgical instrument (300) includes a shaft (310) as previously described.

The blade portion (320) extends from the shaft (310) along a central axis (A) to a blade tip (322), and includes a pair of symmetrical side edge portions (324) with a cutting edge (360) defined on one side edge portion (324A).

The cutting edge (360) is oriented at a blade angle (α) of about 12.5° relative to the central axis (A) and extends to the blade tip (322) such that the cutting edge (360) at least partially faces towards the blade tip (322) to facilitate incision formation when the blade portion (320) is advanced into the eye in a direction parallel with the central axis (A).

The proximal end of the blade portion (320) extends at least partially about a circumference of the distal end (312) of the shaft (310). Advantageously, this enables the distal end (312) of the shaft (310) to be readily inserted into the incision as the blade portion (320) is advanced into the eye in a direction parallel with the central axis (A).

Further, both side edge portions (324) gently taper or converge toward the blade tip (322) in a linear direction from the proximal end. Accordingly, the proximal end is the widest part or apex (B:B) of the blade portion (320) and has a maximum width of about 0.6mm.

In the figure shown, the proximal end (312) of the shaft (310) is connectable to a cannula by way of a friction fit.

Referring to Figure 14, the instrument (300) shown in Figure 13 is shown in the form of a cannula (1010) having a male Luer-Lok^{™} connection fitting (800) for connecting to a syringe containing the viscoelastic fluid, for example.

The male Luer-Lok^{™} connection fitting (800) is overmoulded over the proximal end (312) of the shaft (310). Further, the shaft (310) including distal portion angled relative to a proximal portion at an angle of about 135°.

In the present specification and claims (if any), the word *'comprising'* and its derivatives including *'comprises'* and *'comprise'* include each of the stated integers but does not exclude the inclusion of one or more further integers.

In compliance with the statute, the invention has been described in language more or less specific to structural or methodical features. It is to be understood that the invention is not limited to specific features shown or described since the means herein described comprises preferred forms of putting the invention into effect. The invention is, therefore, claimed in any of its forms or modifications within the proper scope of the appended claims appropriately interpreted by those skilled in the art.

## Claims

1. A multi-purpose ophthalmic blade (100) adapted for cataract extraction surgery, said blade (100) including:
a shaft (110) connectable to a handle (900);
a first blade portion (130) extending from the shaft (110) along a central axis (A), said first blade portion (130) including a pair of opposed side edge portions (132) extending about the central axis (A), the side edge portions (132) defining a first pair of opposed cutting edges (160) each oriented at a first angle relative to said central axis (A) and that at least partially converge towards a distal end of the first blade portion (130); and
a second blade portion (140) extending distally forward from the first blade portion (130) along the central axis (A) to a blade tip (122), said second blade portion (140) including a pair of opposed parallel side edge portions (141) and a pair of converging side edge portions (142) extending distally forward from the parallel side edge portions (141), said converging side edge portions (142) defining a second pair of cutting edges (170) that converge to the blade tip (122), said second pair of cutting edges (142) each being oriented at a second angle relative to said central axis (A),
wherein said first blade portion (130) is adapted for making a primary incision and said second blade portion (140) is adapted for making a side port incision, **characterised in that** the pair of opposed parallel side edge portions (141) has no cutting edges defined thereon.

2. The blade (100) of claim 1, wherein the first blade portion (130) is adapted for making the primary incision having a maximum width of 2.2mm and a length of about 4.0mm.

3. The blade (100) of claim 1 or claim 2, wherein the second blade portion (140) is adapted for making the side portion incision having a maximum width of about 1.0mm and a length of between about 0.7mm and about 1.2mm.

4. The blade (100) of any one of claims 1 to 3, wherein the second blade portion (140) extends distally forward of the first blade portion (130) a distance equal to, or less than, a multiple of 1.2 times a width of the second blade portion (140) at its widest point.

5. The blade (100) of claim 4, wherein the distance is equal to, or less than, a multiple of 1.1 times the width of the second blade portion (140) at its widest point.

6. The blade (100) of claim 4, wherein the distance is equal to, or less than, a multiple of 1.0 times the width of the second blade portion (140) at its widest point.

7. The blade (100) of claim 4, wherein the distance is equal to a multiple of between 0.7 and 1.0 times the width of the second blade portion (140) at its widest point.

8. The blade (100) of any one of claims 1 to 7, wherein the blade (100) includes a body (120) defining the first blade portion (130) and the second blade portion (140) and wherein the body (120) is in the form of a plate.

9. The blade (100) of claim 8, wherein the body (120) defines the first blade portion (130) at or near a proximal end of the body (120), the second blade portion (140) at or near the distal end of the body (120) and a junction (150) between the first blade portion (130) and the second blade portion (140).

10. The blade (100) of claim 9, wherein the pair of opposed side edge portions (132) of the first blade portion (130) flare outwards away from the central axis (A) to define a widest part or portion of the blade (100) and then taper towards the central axis (A) at or near the junction (150) as they extend between the proximal end and the junction (150).

11. The blade (100) of claim 10, wherein the side edge portions (132) gently taper in a linear direction from the widest part or portion to the junction (150) to define distal-end facing side edge portions (136) having the first pair of cutting edges (160) defined thereon so as to form the primary incision when the first blade portion (130) is advanced into the eye in a direction parallel with the central axis (A).

12. The blade (100) of claim 11, wherein the distal-end facing side edge portions (136) are oriented at the first angle relative to the central axis (A).

13. The blade (100) of any one of claims 1 to 12, wherein the first angle is between about 25° and about 35°.

14. The blade (100) of any one of claims 1 to 13, wherein the second angle is between about 30° and about 60°.

15. The blade (100) of any one of claims 1 to 14, wherein the blade (100) includes a first marking extending laterally across an upper surface (124) of the blade (100) in a direction substantially perpendicular to the central axis (A) to indicate a suitable depth of insertion for forming the primary incision and a second marking extending laterally across the upper surface (124) of the blade (100) in a direction substantially perpendicular to the central axis (A) to indicate a depth of insertion for forming the side port incision.

## Patentansprüche

1. Multifunktionale ophthalmologische Klinge (100), die für die Kataraktextraktionschirurgie ausgelegt ist,
wobei die Klinge (100) Folgendes beinhaltet:
einen Schaft (110), der mit einem Griff (900) verbindbar ist;
einen ersten Klingenabschnitt (130), der sich von dem Schaft (110) entlang einer Mittelachse (A) erstreckt, wobei der erste Klingenabschnitt (130) ein Paar von gegenüberliegenden Seitenkantenabschnitten (132) beinhaltet, die sich um die Mittelachse (A) erstrecken, wobei die Seitenkantenabschnitte (132) ein erstes Paar von gegenüberliegenden Schneidkanten (160) definieren, die jeweils in einem ersten Winkel relativ zu der Mittelachse (A) ausgerichtet sind und die zumindest teilweise zu einem distalen Ende des ersten Klingenabschnitts (130) hin konvergieren; und
einen zweiten Klingenabschnitt (140), der sich von dem ersten Klingenabschnitt (130) entlang der Mittelachse (A) distal nach vorne zu einer Klingenspitze (122) erstreckt, wobei der zweite Klingenabschnitt (140) ein Paar von gegenüberliegenden parallelen Seitenkantenabschnitten (141) und ein Paar von konvergierenden Seitenkantenabschnitten (142), die sich von den parallelen Seitenkantenabschnitten (141) distal nach vorne erstrecken, beinhaltet, wobei die konvergierenden Seitenkantenabschnitte (142) ein zweites Paar von Schneidkanten (170) definieren, die zu der Klingenspitze (122) konvergieren, wobei das zweite Paar von Schneidkanten (142) jeweils in einem zweiten Winkel relativ zu der Mittelachse (A) ausgerichtet ist,
wobei der erste Klingenabschnitt (130) ausgelegt ist, um einen primären Einschnitt vorzunehmen, und der zweite Klingenabschnitt (140) ausgelegt ist, um einen Seitenöffnungseinschnitt vorzunehmen, **dadurch gekennzeichnet, dass** das Paar von gegenüberliegenden parallelen Seitenkantenabschnitten (141) keine daran definierten Schneidkanten aufweist.

2. Klinge (100) nach Anspruch 1, wobei der erste Klingenabschnitt (130) ausgelegt ist, um den primären Einschnitt mit einer maximalen Breite von 2,2 mm und einer Länge von etwa 4,0 mm vorzunehmen.

3. Klinge (100) nach Anspruch 1 oder Anspruch 2, wobei der zweite Klingenabschnitt (140) ausgelegt ist, um den Seitenabschnitteinschnitt mit einer maximalen Breite von etwa 1,0 mm und einer Länge zwischen etwa 0,7 mm und etwa 1,2 mm vorzunehmen.

4. Klinge (100) nach einem der Ansprüche 1 bis 3, wobei sich der zweite Klingenabschnitt (140) distal vor dem ersten Klingenabschnitt (130) um einen Abstand erstreckt, der gleich oder kleiner als ein Vielfaches des 1,2-fachen einer Breite des zweiten Klingenabschnitts (140) an seiner breitesten Stelle ist.

5. Klinge (100) nach Anspruch 4, wobei der Abstand gleich oder kleiner als ein Vielfaches des 1,1-fachen der Breite des zweiten Klingenabschnitts (140) an seiner breitesten Stelle ist.

6. Klinge (100) nach Anspruch 4, wobei der Abstand gleich oder kleiner als ein Vielfaches des 1,0-fachen der Breite des zweiten Klingenabschnitts (140) an seiner breitesten Stelle ist.

7. Klinge (100) nach Anspruch 4, wobei der Abstand gleich einem Vielfachen des 0,7- bis 1,0-fachen der Breite des zweiten Klingenabschnitts (140) an seiner breitesten Stelle ist.

8. Klinge (100) nach einem der Ansprüche 1 bis 7, wobei die Klinge (100) einen Körper (120) beinhaltet, der den ersten Klingenabschnitt (130) und den zweiten Klingenabschnitt (140) definiert, und wobei der Körper (120) die Form einer Platte aufweist.

9. Klinge (100) nach Anspruch 8, wobei der Körper (120) den ersten Klingenabschnitt (130) an oder in der Nähe eines proximalen Endes des Körpers (120), den zweiten Klingenabschnitt (140) an oder in der Nähe des distalen Endes des Körpers (120) und eine Verbindung (150) zwischen dem ersten Klingenabschnitt (130) und dem zweiten Klingenabschnitt (140) definiert.

10. Klinge (100) nach Anspruch 9, wobei sich das Paar von gegenüberliegenden Seitenkantenabschnitten (132) des ersten Klingenabschnitts (130) nach außen von der Mittelachse (A) weg erweitert, um einen breitesten Teil oder Abschnitt der Klinge (100) zu definieren, und sich dann zu der Mittelachse (A) hin an oder in der Nähe der Verbindung (150) verjüngt, wenn sie sich zwischen dem proximalen Ende und der Verbindung (150) erstrecken.

11. Klinge (100) nach Anspruch 10, wobei sich die Seitenkantenabschnitte (132) in einer linearen Richtung von dem breitesten Teil oder Abschnitt zu der Verbindung (150) leicht verjüngen, um zum distalen Ende weisende Seitenkantenabschnitte (136) zu definieren, auf denen das erste Paar von Schneidkanten (160) definiert ist, um den primären Einschnitt zu bilden, wenn der erste Klingenabschnitt (130) in eine Richtung parallel zu der Mittelachse (A) in das Auge vorgeschoben wird.

12. Klinge (100) nach Anspruch 11, wobei die zum distalen Ende weisenden Seitenkantenabschnitte (136) in dem ersten Winkel relativ zu der Mittelachse (A) ausgerichtet sind.

13. Klinge (100) nach einem der Ansprüche 1 bis 12, wobei der erste Winkel zwischen etwa 25° und etwa 35° liegt.

14. Klinge (100) nach einem der Ansprüche 1 bis 13, wobei der zweite Winkel zwischen etwa 30° und etwa 60° liegt.

15. Klinge (100) nach einem der Ansprüche 1 bis 14, wobei die Klinge (100) eine erste Markierung, die sich seitlich über eine obere Oberfläche (124) der Klinge (100) in einer Richtung im Wesentlichen senkrecht zu der Mittelachse (A) erstreckt, um eine geeignete Einführtiefe zum Bilden des primären Einschnitts anzugeben, und eine zweite Markierung, die sich seitlich über die obere Oberfläche (124) der Klinge (100) in einer Richtung im Wesentlichen senkrecht zu der Mittelachse (A) erstreckt, um eine Einführtiefe zum Bilden des Seitenöffnungseinschnitts anzugeben, beinhaltet.

## Revendications

1. Instrument chirurgical ophtalmique polyvalent (100) adapté à la chirurgie d'extraction de la cataracte, ledit instrument ophtalmique (100) comprenant :
un arbre (110) pouvant être relié à une poignée (900) ;
une première portion d'instrument chirurgical (130) s'étendant depuis l'arbre (110) le long de l'axe central (A), ladite première portion d'instrument chirurgical (130) comprenant une paire de portions de bord latéral opposées (132) s'étendant autour de l'axe central (A), les portions de bord latéral (132) définissant une première paire d'arêtes de coupe opposés (160) orientés chacun suivant un premier angle par rapport audit axe central (A) et qui convergent au moins partiellement en direction de l'extrémité distale de la première portion d'instrument chirurgical (130) ; et
une seconde portion d'instrument chirurgical (140) s'étendant de manière distale vers l'avant à partir de la première portion d'instrument chirurgical (130) le long de l'axe central (A) jusqu'à une pointe d'instrument chirurgical (122), ladite seconde portion d'instrument chirurgical (140) comprenant une paire de portions de bord latéral parallèles opposées (141) et une paire de portions de bord latéral convergentes (142) s'étendant de manière distale vers l'avant à partir des portions de bord latéral parallèles (141), lesdites portions de bord latéral convergentes (142) définissant une seconde paire d'arêtes de coupe (170) qui convergent vers la pointe d'instrument chirurgical (122), ladite seconde paire d'arêtes de coupe (142) étant chacune orientée suivant un second angle par rapport audit axe central (A),
ladite première portion d'instrument chirurgical (130) étant adaptée à la réalisation d'une incision primaire et ladite seconde portion d'instrument chirurgical (140) étant adaptée à la réalisation d'une incision d'orifice latéral, **caractérisé en ce que** la paire de portions de bord latéral parallèles opposées (141) ne comporte pas sur elle d'arêtes de coupe définies.

2. Instrument chirurgical (100) selon la revendication 1, ladite première portion d'instrument chirurgical (130) étant adaptée à la réalisation de l'incision primaire comportant une largeur maximale de 2,2 mm et une longueur d'environ 4,0 mm.

3. Instrument chirurgical (100) selon la revendication 1 ou la revendication 2, ladite seconde portion d'instrument chirurgical (140) étant adaptée à la réalisation de l'incision de la portion latérale comportant une largeur maximale d'environ 1,0 mm et une longueur comprise entre environ 0,7 mm et environ 1,2 mm.

4. Instrument chirurgical (100) selon l'une quelconque des revendications 1 à 3, ladite seconde portion d'instrument chirurgical (140) s'étendant de manière distale vers l'avant de la première portion d'instrument chirurgical (130) sur une distance inférieure ou égale à un multiple de 1,2 fois la largeur de la seconde portion d'instrument chirurgical (140) à son point le plus large.

5. Instrument chirurgical (100) selon la revendication 4, ladite distance étant inférieure ou égale à un multiple de 1,1 fois la largeur de la seconde portion d'instrument chirurgical (140) au niveau de son point le plus large.

6. Instrument chirurgical (100) selon la revendication 4, ladite distance étant inférieure ou égale à un multiple de 1,0 fois la largeur de la seconde portion d'instrument chirurgical (140) à son point le plus large.

7. Instrument chirurgical (100) selon la revendication 4, ladite distance étant égale à un multiple compris entre 0,7 et 1,0 fois la largeur de la seconde portion d'instrument chirurgical (140) au niveau de son point le plus large.

8. Instrument chirurgical (100) selon l'une quelconque des revendications 1 à 7, ledit instrument chirurgical (100) comprenant un corps (120) définissant la première portion d'instrument chirurgical (130) et la seconde portion d'instrument chirurgical (140) et le corps (120) se présentant sous la forme d'une plaque.

9. Instrument chirurgical (100) selon la revendication 8, ledit corps (120) définissant la première portion d'instrument chirurgical (130) au niveau ou à proximité de l'extrémité proximale du corps (120), ladite seconde portion d'instrument chirurgical (140) au niveau ou à proximité de l'extrémité distale du corps (120) et une jonction (150) entre la première portion d'instrument chirurgical (130) et la seconde portion d'instrument chirurgical (140).

10. Instrument chirurgical (100) selon la revendication 9, ladite paire de portions de bord latéral opposées (132) de la première portion d'instrument chirurgical (130) s'évasant vers l'extérieur en s'éloignant de l'axe central (A) de manière à définir la partie ou la portion la plus large de l'instrument chirurgical (100) et s'effilant ensuite en direction de l'axe central (A) au niveau ou à proximité de la jonction (150) lorsqu'elles s'étendent entre l'extrémité proximale et la jonction (150).

11. Instrument chirurgical (100) selon la revendication 10, lesdites portions de bord latéral (132) s'effilant doucement suivant une direction linéaire depuis la partie ou la portion la plus large jusqu'à la jonction (150) de manière à définir des portions de bord latéral faisant face à l'extrémité distale (136) sur lesquelles est définie la première paire d'arêtes de coupe (160) de manière à former l'incision primaire lorsque la première portion d'instrument chirurgical (130) est avancée dans l'œil suivant une direction parallèle à l'axe central (A).

12. Instrument chirurgical (100) selon la revendication 11, lesdites portions de bord latéral faisant face à l'extrémité distale (136) étant orientées suivant le premier angle par rapport à l'axe central (A).

13. Instrument chirurgical (100) selon l'une quelconque des revendications 1 à 12, ledit premier angle étant compris entre environ 25° et environ 35°.

14. Instrument chirurgical (100) selon l'une quelconque des revendications 1 à 13, ledit second angle étant compris entre environ 30° et environ 60°.

15. Instrument chirurgical (100) selon l'une quelconque des revendications 1 à 14, ledit instrument chirurgical (100) comprenant un premier repère s'étendant latéralement à travers une surface supérieure (124) de l'instrument chirurgical (100) suivant une direction sensiblement perpendiculaire à l'axe central (A) de manière à indiquer la profondeur d'insertion appropriée pour former l'incision primaire et un second repère s'étendant latéralement à travers la surface supérieure (124) de l'instrument chirurgical (100) suivant une direction sensiblement perpendiculaire à l'axe central (A) de manière à indiquer la profondeur d'insertion pour former l'incision d'orifice latéral.
